# EUROPEAN PATENT APPLICATION

(11) **EP 1 557 408 A1**
(43) Date of publication of application: **27.07.2005**
(21) Application number: 03758924.9
(22) Date of filing: 27.10.2003
(51) Int. Cl.: C07C 317/22, B41M 5/30

(54) **RECORDING MATERIAL COMPRISING DIPHENYL SULFONE DERIVATIVE AND NOVEL DIPHENYL SULFONE DERIVATIVE COMPOUND**

(30) Priority: 30.10.2002 JP 2002315812
(71) Applicant: NIPPON SODA CO., LTD., Chiyoda-ku, Tokyo 100-8165 (JP)
(72) Inventor: HIDAKA, Tomoya, Nippon Soda Co., Ltd.,, Ichihara-shi, Chiba 290-0045 (JP); FUJII, Hiroshi, Nippon Soda Co., Ltd., Ichihara-shi, Chiba 290-0045 (JP); SATO, Shinichi, Nippon Soda Co., Ltd., Ichihara-shi, Chiba 290-0045 (JP)
(74) Representative: Williams, Aylsa
(86) International application number: PCT/JP2003/013689
(87) International publication number: WO 2004/039770

(57) **Abstract**

The present invention provides a recording material which has excellent background stability, specifically heat resistance and heat and humidity resistance of the background. The recording material containing a color forming dye is characterized by containing at least one kind of diphenylsulfone derivative represented by the general formula (I): 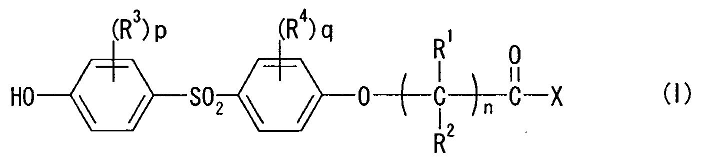 (wherein R¹ and R² each represents a hydrogen atom or a C1 to C6 alkyl group, n represents an integer of 1 to 6, R³ and R⁴ each represents a halogen atom, a C1 to C6 alkyl group, etc., p and q each represents an integer of 0 to 4, X represents OR⁵ or NR⁶R⁷ wherein R⁵ represents a C1 to C6 alkyl group, a C1 to C6 hydroxyalkyl group, or a C1 to C6 alkoxy-C1 to C6 alkyl group, and R⁶ and R⁷ each represents a hydrogen atom, a C1 to C6 alkyl group, etc).

## Description

### Technical Field

The present invention relates to a recording material containing a diphenyl sulfone derivative which has excellent background stability, specifically in heat resistance and heat and humidity resistance of the background part, and novel diphenyl sulfone derivative compounds.

### Background Art

Because recording materials utilizing color-development caused by reaction between color forming dyes and developers can record in a moment by means of relatively simple apparatuses without complicated treatments such as development and fixation, they are widely used for heat-sensitive recording paper to register outputs from facsimiles, printers, or the like, pressure-sensitive copying paper for forms to copy several sheets at the same time, or the like. As these recording materials, recording materials which have excellent heat resistance and heat and humidity resistance of background parts are specifically required, in view of the efficiency for drying the heat-sensitive paper at high temperature to shorten its drying time, or, in view of storage conditions at high temperature or various usage conditions, in addition to those which can be color-developed quickly, with maintining whiteness in the portion with undeveloped color (hereinafter referred to as "a background part"), or with producing color-developed images having high durabilities. Therefore, efforts have been expended on developments of color forming dyes, developers, image stabilizers, and the like, but those having good and fully satisfying balances among dynamic sensitivities, background and image stabilities, and the like, have not yet been found.

Though 4-isopropoxy-4'-hydroxy diphenylsulfone is known in the prior art as a recording material which has excellent dynamic sensitivity, its background stability is not yet satisfactory.

Moreover, compounds similar to those of the present invention are disclosed (for example, see Patent literatures 1 to 5).

### Patent literature 1:

Japanese Unexamined Patent Application, First Publication No. Sho 61-98350 (page 11 to 12)

### Patent literature 2:

Japanese Unexamined Patent Application, First Publication No. Sho 61-110136 (page 4)

### Patent literature 3:

Japanese Unexamined Patent Application, First Publication No. Hei 3-33844 (page 4)

### Patent literature 4:

Japanese Unexamined Patent Application, First Publication No. Hei 3-149545 (page 7)

### Patent literature 5:

International Unexamined Patent Application, First Publication No. 01/16097 (page 67 to 68)

Compounds described in the above-mentioned Patent literatures 1 to 4 are used as silver halide color photo-sensitive materials, and compounds described in Patent literature 5 are used as starting materials, but uses as developers of recording materials are not known.

### Disclosure of Invention

An object of the present invention is to solve problems in the conventional recording materials as described above, and to provide a recording material which has excellent background stability, specifically in heat resistance and moisture heat resistance of the background part, and novel diphenylsulfone derivative compounds.

That is, the first invention of the present invention provides a recording material containing a color forming dye, characterized by containing at least one kind of diphenylsulfone derivatives represented by the general formula (I): 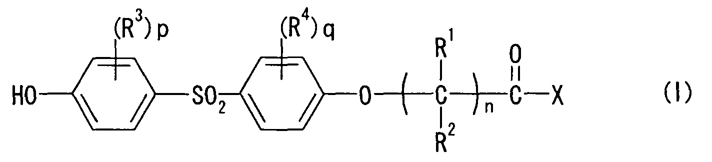 (wherein R¹ and R² each independently represents a hydrogen atom or a C1 to C6 alkyl group, n represents an integer of 1 to 6, R³ and R⁴ each independently represents a halogen atom, a C1 to C6 alkyl group, a C2 to C6 alkenyl group, or a C1 to C6 alkoxy group, p and q each independently represents an integer of 0 to 4, R³ and R⁴ may respectively be the same or different when p and q each represents an integer of 2 or more, X represents OR⁵ or NR⁶R⁷, R⁵ represents a C1 to C6 alkyl group, a C1 to C6 hydroxyalkyl group, a C1 to C6 alkoxy-C1 to C6 alkyl group, a phenoxy-C1 to C6 alkyl group, a phenyl group which may have a substituent, or a benzyl group which may have a substitutent, and R⁶ and R⁷ each independently represents a hydrogen atom, a C1 to C6 alkyl group, a phenyl group which may have a substituent, or a benzyl group which may have a substituent).

Moreover, the second invention of the present invention provides diphenylsulfone derivatives represented by the general formula (II): 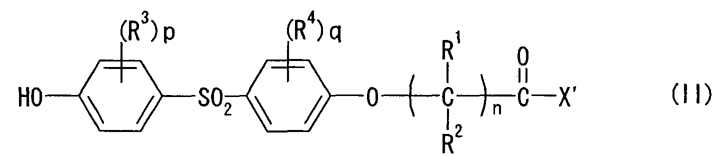 (wherein R¹ and R² each independently represents a hydrogen atom or a C1 to C6 alkyl group, n represents an integer of 1 to 6, R³ and R⁴ each independently represents a halogen atom, a C1 to C6 alkyl group, a C2 to C6 alkenyl group, or a C1 to C6 alkoxy group, p and q each independently represents an integer of 0 to 4, R³ and R⁴ may respectively be the same or different when p and q each represents an integer of 2 or more, X' represents OR⁸ or NR⁶R⁷, R⁸ represents a C1 to C6 alkyl group, a C1 to C6 hydroxyalkyl group, a C1 to C6 alkoxy-C1 to C6 alkyl group, a phenoxy-C1 to C6 alkyl group, a phenyl group which may have a substituent, or a benzyl group which may have a substitutent, and R⁶ and R⁷ each independently represents a hydrogen atom, a C1 to C6 alkyl group, a phenyl group which may have a substituent, or a benzyl group which may have a substituent, provided, however, that R⁸ does not represent an ethyl group, when R¹ represents a hydrogen atom, R² represents a hydrogen atom, n represents 1, p represents 0, q represents 0, X' represents OR⁸).

In the following, the present invention will be explained in detail.

In the general formula (I) and (II), R¹ and R² each independently represents a hydrogen atom or a C1 to C6 alkyl group such as a methyl group, an ethyl group, an n-propyl group, an isopropyl group, an n-butyl group,a sec-butyl group, a t-butyl group, an n-pentyl group, an isopentyl group, a neopentyl group, a t-pentyl group, an n-hexyl group, an isohexyl group, a 1-methylpentyl group, or a 2-methylpentyl group.

R³ and R⁴ each independently represents a halogen atom such as a fluorine atom, a chlorine atom, a bromine atom, or an iodine atom; a C1 to C6 alkyl group such as a methyl group, an ethyl group, an n-propyl group, an isopropyl group, an n-butyl group, a sec-butyl group, a t-butyl group, an n-pentyl group, an iso-pentyl group, a neopentyl group, a t-pentyl group, an n-hexyl group, an isohexyl group, a 1-methylpentyl group, or a 2-methylpentyl group; a C2 to C6 alkenyl group such as a vinyl group, an allyl group, an isopropenyl group, a 1-propenyl group, a 2-butenyl group, a 3-butenyl group, a 1,3-butanedienyl group, a 2-methyl-2-propenyl group, a 1-pentenyl group, a 2-pentenyl group, a 3-pentenyl group, a 4-pentenyl group, a 1-hexenyl group, a 2-hexenyl group, or a 3-hexenyl group; or a C1 to C6 alkoxy group such as a methoxy group, an ethoxy group, an n-propoxy group, an isopropoxy group, an n-butoxy group, a sec-butoxy group, or a t-butoxy group.

R⁵ and R⁸ each independently represents a C1 to C6 alkyl group such as a methyl group, an ethyl group, an n-propyl group, an isopropyl group, an n-butyl group, a sec-butyl group, a t-butyl group, an n-pentyl group, an isopentyl group, a neopentyl group, a t-pentyl group, an n-hexyl group, an isohexyl group, a 1-methylpentyl group, or a 2-methylpentyl group; a C1 to C6 hydroxyalkyl group such as a hydroxymethyl group, a 1-hydroxyethyl group, or 2-hydroxyethyl group; a C1 to C6 alkoxy C1 to C6 alkyl group such as a methoxymethyl group, a methoxyethyl group, an ethoxymethyl group, or an ethoxyethyl group; a phenoxy C1 to C6 alkyl group such as a phenoxymethyl group, or a phenoxyethyl group; a phenyl group which may have a substitutent, or a benzyl group which may have a substitutent benzyl, and R⁶ and R⁷ each independently represents a hydrogen atom; a C1 to C6 alkyl group such as a methyl group, an ethyl group, an n-propyl group, an isopropyl group, an n-butyl group, a sec-butyl group, a t-butyl group, an n-pentyl group, an isopentyl group, a neopentyl group, a t-pentyl group, an n-hexyl group, an isohexyl group, a 1-methylpentyl group, or a 2-methylpentyl group; a phenyl group which may have a substitutent, or a benzyl group which may have a substitutent. Moreover, halogen atoms such as a fluorine atom, a chlorine atom, a bromine atom, and an iodine atom; C1 to C6 alkyl groups such as a methyl group, an ethyl group, an n-propyl group, an isopropyl group, an n-butyl group, a sec-butyl group, a t-butyl group, an n-pentyl group, an isopentyl group, a neopentyl group, a t-pentyl group, an n-hexyl group, an isohexyl group, a 1-methylpentyl group, and a 2-methylpentyl group; C2 to C6 alkenyl groups such as a vinyl group, an allyl group, an isopropenyl group, a 1-propenyl group, a 2-butenyl group, a 3-butenyl group, a 1,3-butanedienyl group, a 2-methyl-2-propenyl group, a 1-pentenyl group, a 2-pentenyl group, a 3-pentenyl group, a 4-pentenyl group, a 1-hexenyl group, a 2-hexenyl group, and a 3-hexenyl group; C1 to C6 alkoxy groups such as a methoxy group, an ethoxy group, an n-propoxy group, an isopropoxy group, an n-butoxy group, a sec-butoxy group, and a t-butoxy group can be mentioned as the substitutents of the phenyl group and the benzyl group of R⁵, R⁶, R⁷ and R⁸ described above. These substituents may exist in plurality, and, if so, each of the substituents may be the same or different.

However, in the general formula (II), R⁸ does not represent an ethyl group, when R¹ represents a hydrogen atom, R² represents a hydrogen atom, n represents 1, p represents 0, q represents 0, and X' represents OR⁸.

The compound represented by the general formula (I) for using in the present invention can be prepared by being reacted a compound represented by the general formula (III): 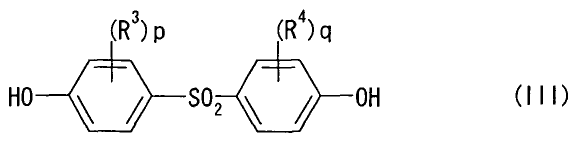 (wherein R³, R⁴, p, and q represent the same meanings as described above) with a compound represented by the general formula (IV): 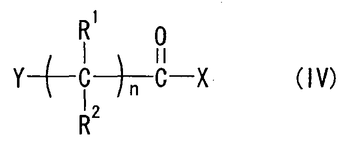 (wherein R¹, R², X, and n represent the same meanings as described above, and Y represents a halogen atom such as a chlorine atom or a bromine atom) in a solvent in the presence of bases.

As the above-mentioned solvent, water; amides such as N,N-dimethylformamide, and N,N-dimethylacetamide; sulfoxides such as dimethylsulfoxide; nitriles such as acetonitrile; alcohols such as methanol, ethanol, n-propanol, and isopropanol; ketones such as acetone, and methyl isobutyl ketone; aromatic hydrocarbons such as benzene, toluene, xylene, and mesitylene, and the like can be mentioned. These solvents may be used alone or as a mixture of two or more.

As the above-mentioned bases, alkali metal hydroxides such as sodium hydroxide, and potassium hydroxide; alkaline earth metal hydroxides such as magnesium hydroxide, and calcium hydroxide; alkali metal carbonates such as sodium carbonate, potassium carbonate, calcium carbonate, and sodium hydrogencarbonate, amines such as triethylamine, and pyridine, and the like can be mentioned. These bases may be used alone or as a mixture of two or more.

The compounds which can be synthesized as described above are listed in Tables 1 and 2.

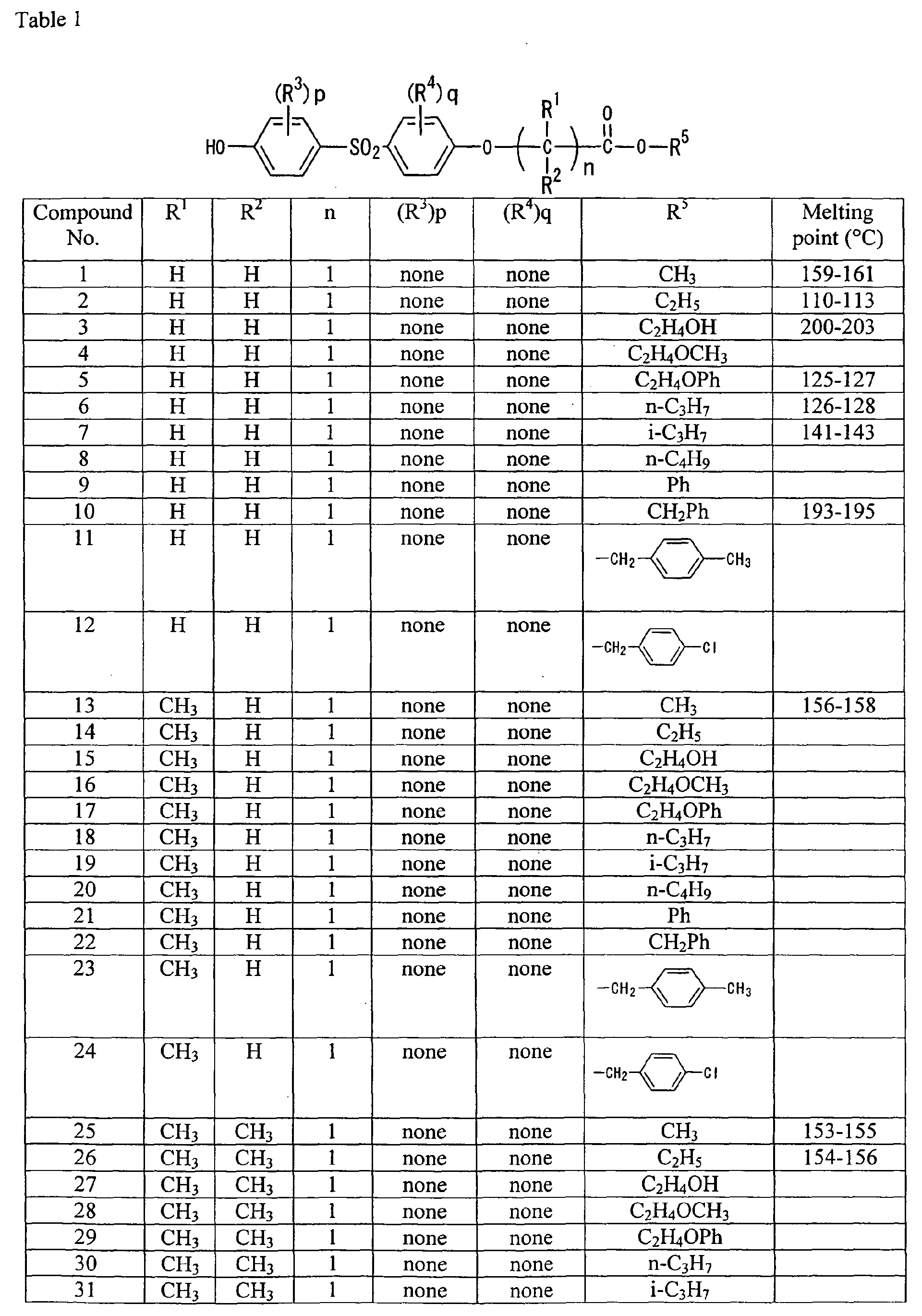

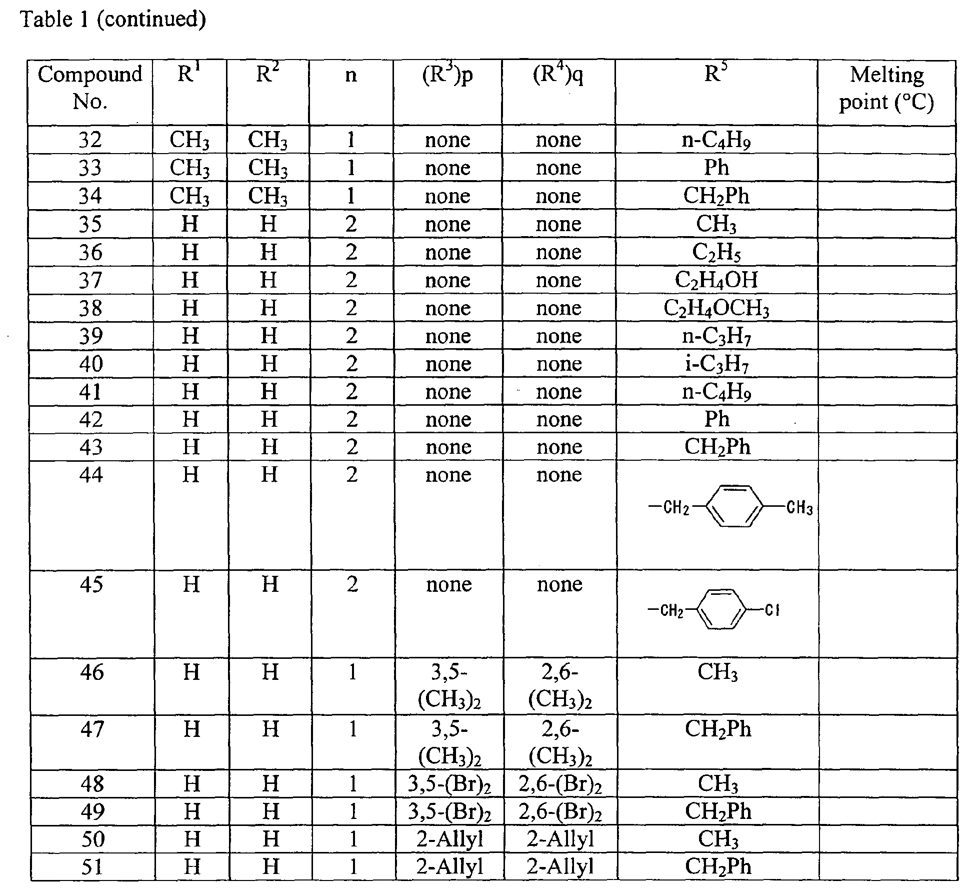

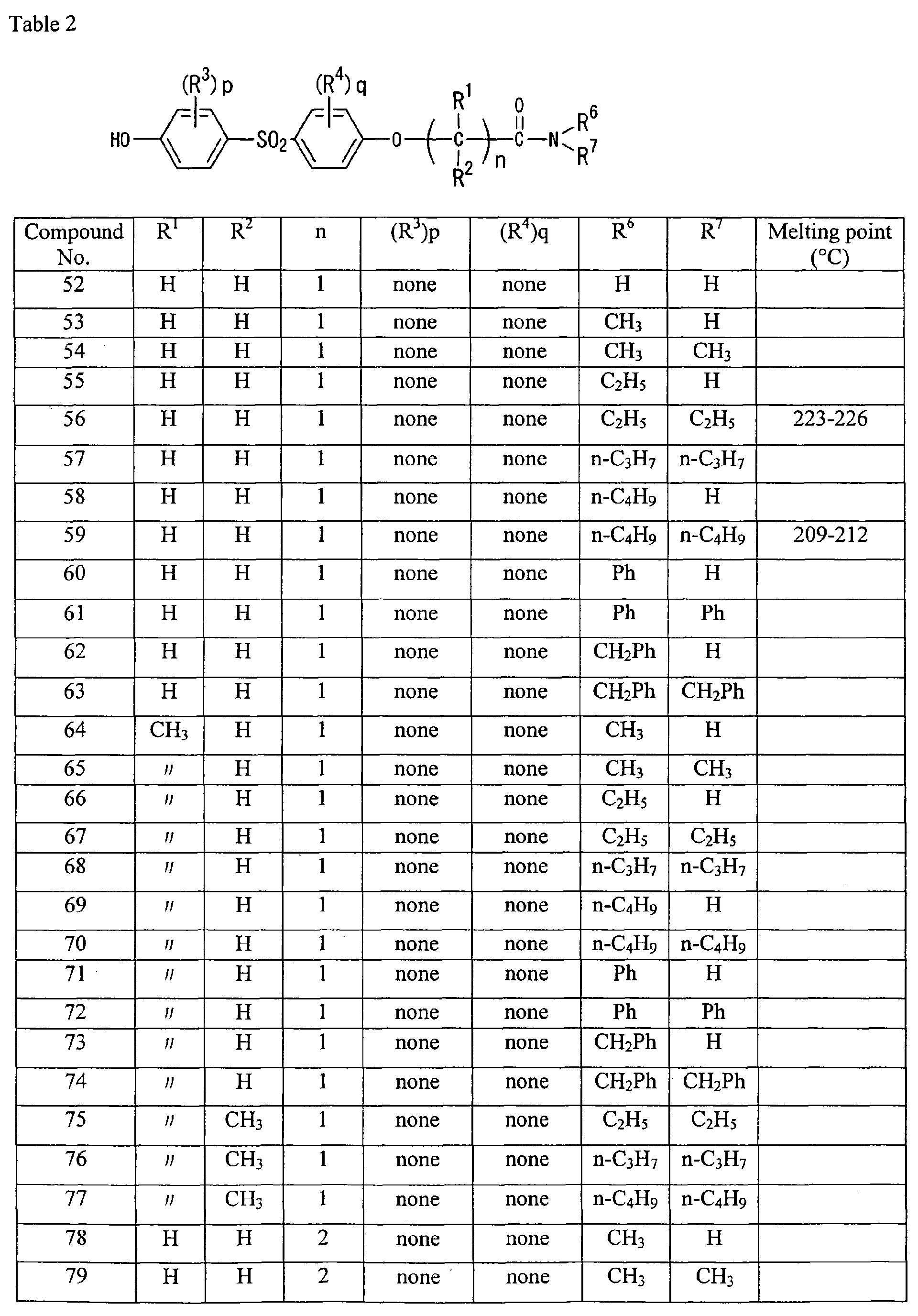

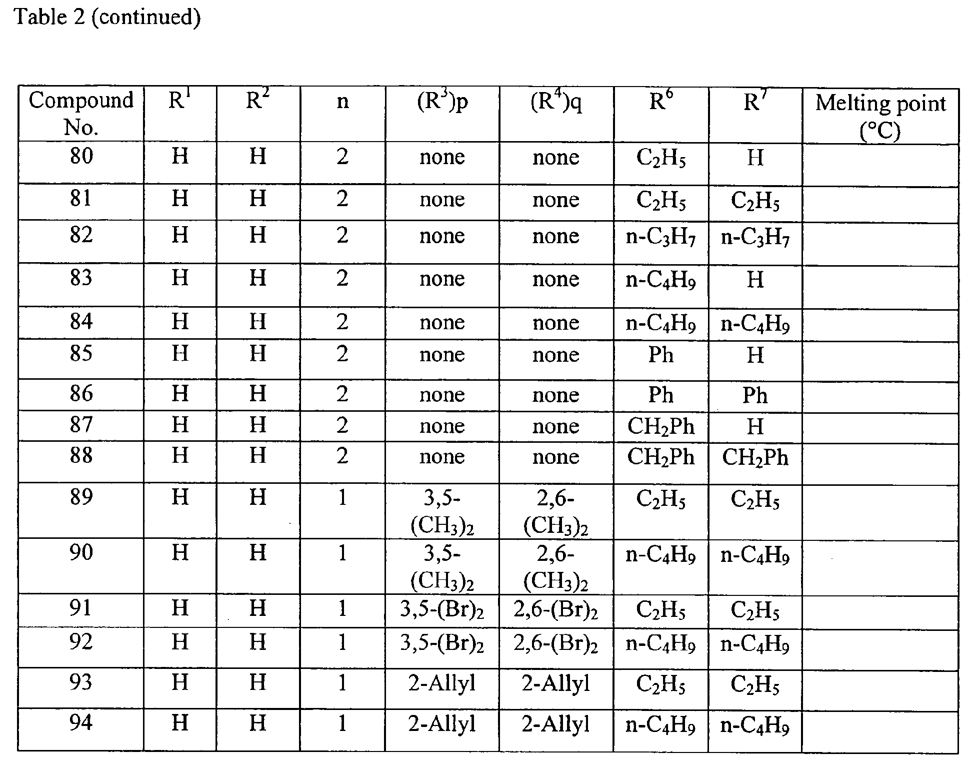

The present invention may be applied to any uses of recording materials using color forming dyes, for example, to heat-sensitive recording materials such as heat-sensitive recording paper or pressure-sensitive copying materials such as pressure-sensitive copying paper.

When this invention is used for the heat-sensitive recording paper, it may be used in a manner similar to that of a known developer. For example, the heat-sensitive recording paper can be produced by mixing together with suspensions, each of which contains microparticles of the compound of the present invention or microparticles of the color forming dye, respectively dispersed in an aqueous solution of a water-soluble binder such as polyvinyl alcohol or cellulose, followed by coating the mixed suspensions on a supporting substrate such as paper or the like, and then by drying the coated suspensions.

The amount of the compound represented by the general formula (I) used is 1 to 10 parts by weight, preferably 1.5 to 5 parts by weight, with respect to one part by weight of the color forming dye.

In the recording material of the present invention, one or more kinds of known developers, image stabilizers, sensitizers, fillers, dispersants, antioxidants, desensitizers, surface lubricants, antifoaming agents, photostabilizers, fluorescent whitening agents, and the like can be contained as needed, in addition to the color forming dye and the compound represented by the general formula (I).

These agents may be included not only in a color forming layer, but also in an optional layer such as a protective layer or the like if a multi-layer structure is applied. In particular, when an overcoat layer and/or an undercoat layer is provided on top of and/or at the bottom of the color forming layer, antioxidants, photostabilizers, and the like may be included in the layers. In addition, the antioxidants and the photostabilizers may be included in the layers in the form of being contained in microcapsules as needed.

As the color forming dye used for the recording material of the present invention, leuco dyes such as fluoran bases, phthalide bases, lactam bases, triphenylmethane bases, phenothiazine bases, and spiropyran bases can be mentioned; however, the color forming dyes are not limited to these, and optional color forming dyes which can be color-developed by contacting with developers which are acid agents can be used. Moreover, without mentioning that these color forming dyes can be used alone for preparing recording materials of their color forming colors, these can also be used by mixing two or more. For example, color forming dyes of three primary colors, that is, red, blue, and green, or a black color forming dye can be used by mixing together for preparing recording materials which can develop deep black.

As the color forming dyes of fluoran bases, for example, 3-diethylamino-6-methyl-7-anilinofluoran, 3-dibutylamino-6-methyl-7-anilinofluoran, 3-(N-ethyl-N-isobutylamino)-6-methyl-7-anilinofluoran, 3-(N-methyl-N-propylamino)-6-methyl-7anilinofluoran, 3-(N-ethyl-N-isopentylamino)-6-methyl-7-anilinofluoran, 3-diethylamino-7-(o-chloroanilino)fluoran, 3-dibutylamino-7-(o-chloroanilino)fluoran, 3-(N-ethyl-p-toluidino)-6-methyl-7-anilinofluoran, 3-(N-cyclohexyl-N-methylamino)-6-methyl-7-anilinofluoran, 3-pyrrolidino-6-methyl-7-anilinofluoran, 3-piperidino-6-methyl-7-anilinofluoran, 3-dimethylamino-7-(m-trifluoromethylanilino)fluoran, 3-dipentylamino-6-methyl-7-anilinofluoran, 3-(N-ethoxypropyl-N-ethylamino)-6-methyl-7-anilinofluoran, 3-dibutylamino-7-(o-fluoroanilino)fluoran, 3-diethylaminobenzo[a]fluoran, 3-dimethylamino-6-methyl-7-chlorofluoran, 3-diethylamino-5-methyl-7-dibenzylaminofluoran, 3-diethylamino-7-dibenzylaminofluoran, 3-diethylamino-5-chlorofluoran, 3-diethylamino-6-(N,N'-dibenzylamino)fluoran, 3,6-dimethoxyfluoran, 2,4-dimethyl-6-(4-dimethylaminophenyl)aminofluoran, and the like can be mentioned.

As near-infrared-ray absorbing dyes, 3-{4-[4-(4-anilino)-anilino]anilino}-6-methyl-7-chlorofluoran, 3,3-bis[2-(4-dimethylaminophenyl)-2-(4-methoxyphenyl)vinyl]-4,5,6,7-tetrachlorophthalide, 3,6,6'-tris(dimethylamino)spiro(fluorine-9,3'-phthalide), and the like can be mentioned.

In addition, 3,3-bis(4'-diethylaminophenyl)-6-diethylaminophthalide can also be mentioned.

As the aforementioned developers, bisphenol compounds such as bisphenol A, 4,4'-sec-butylidenebisphenol, 4,4'-cyclohexylidenebisphenol, 2,2-dimethyl-3,3-bis(4-hydroxyphenyl)butane, 2,2'-dihydroxydiphenyl, pentamethylene-bis(4-hydroxylienzoate), 2,2-dimethyl-3,3-di(4-hydroxyphenyl)pentane, and 2,2-di(4-hydroxyphenyl)hexane; sulfur-containing bisphenol compounds such as 4,4'-dihydroxydiphenylthio ether, 1,7-di(4-hydroxyphenylthio)-3,5-dioxaheptane, 2,2'-bis(4-hydroxyphenylthio)diethyl ether, and 4,4'-dihydroxy-3,3'-dimethyldiphenylthio ether; 4-hydroxybenzoic acid esters such as benzyl 4-hydroxybenzoate, ethyl 4-hydroxybenzoate, propyl 4-hydroxybenzoate, isopropyl 4-hydroxybenzoate, butyl 4-hydroxybenzoate, isobutyl 4-hydroxybenzoate, chlorobenzyl 4-hydroxybenzoate, methylbenzyl 4-hydroxybenzoate, and diphenylmethyl 4-hydroxybenzoate; benzoic acid metallic salts such as zinc benzoate, and zinc 4-nitrobenzoate; salicylic acids such as 4-[2-(4-methoxyphenyloxy)ethyloxy] salicylic acid; salicylic acid metallic salts such as zinc salicylate, and zinc bis[4-(octyloxycarbonylamino)-2-hydroxybenzoate]; hydroxysulfones such as 4,4'-dihydroxy diphenylsulfone, 2,4'-dihydroxy diphenylsulfone, 4-hydroxy-4'-methyl diphenylsulfone, 4-hydroxy-4'-isopropoxy diphenylsulfone, 4-hydroxy-4'-benzyloxy diphenylsulfone, 4-hydroxy-4'-butoxy diphenylsulfone, 4,4'-dihydroxy-3,3'-diallyl diphenylsulfone, 3,4-dihydroxy-4'-methyl diphenylsulfone, and 4,4'-dihydroxy-3,3',5,5'-tetrabromo diphenylsulfone; benzenesulfonamides such as N-(2-hydroxyphenyl)benzenesulfonamide, N-(2-hydroxyphenyl)-p-toluenesulfonamide, N-(2-hydroxyphenyl)-p-ethyl benzenesulfonamide, N-(2-hydroxyphenyl)-p-methoxy benzenesulfonamide, N-(2-hydroxyphenyl)-p-chloro benzenesulfonamide, N-(2-hydroxyphenyl)-p-phenyl benzenesulfonamide, N-(2-hydroxyphenyl)-p-allyl benzenesulfonamide, and N-(2-hydroxyphenyl)-p-benzyl benzenesulfonamide; 4-hydroxyphthalic acid diesters, such as dimethyl 4-hydroxyphthalate, dicyclohexyl 4-hydroxyphthalate, and diphenyl 4-hydroxyphthalate; hydroxynaphthoic acid esters such as 2-hydroxy-6-carboxynaphthalene; trihalomethyl sulfones such as tribromomethyl phenylsulfone; sulfonylureas such as 4,4'-bis(p-toluenesulfonyl aminocarbonylamino)diphenyl methane; hydroxyacetophenone, p-phenylphenol, benzyl 4-hydroxyphenylacetate, p-benzylphenol, hydroquinone-monobenzyl ether, tetracyanoquinodimethanes, 2'-hydroxy-(4-hydroxyphenylthio)acetanilide, 3'-hydroxy-(4-hydroxyphenylthio)acetanilide, 4'-hydroxy-(4-hydroxyphenylthio)acetanilide, 2,4-dihydroxy-2'-methoxybenzanilide, diphenylsulfone-crosslinked compounds represented by the formula (V): 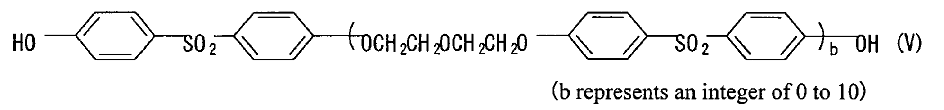 and mixtures thereof can be mentioned.

As the image-stabilizers, for example, diphenylsulfones having an epoxy group such as 4-benzyloxy-4'-(2-methylglycidyloxy)-diphenylsulfone, and 4,4'-diglycidyloxy diphenylsulfone; 1,4-diglycidyloxybenzene, 4-[α-(hydroxymethyl)benzyloxy]-4'-hydroxy diphenylsulfone, 2-propanol derivatives, salicylic acid derivatives, metallic salts (particularly, zinc salts) of oxynaphthoic acid derivatives, metallic salts of 2,2-methylenebis(4,6-t-butylphenyl)phosphate, any other water-insoluble zinc compounds, and the like can be mentioned.

As the sensitizers, for example, higher fatty acid amides such as stearamide, oleamide, N-methylstearamide, erucamide, methylolbehenamide, methylenebis stearamide, and ethylenebis stearamide; higher fatty acid anilides such as stearanilide, and linolanilide; amides such as benzamide, and benzyl amide; anilides such as acetoacetanilide, 4-acetotoluide, salicylanilide, 4-hydroxybenzanilide, and thioacetanilide; dibenzyl oxalate, di(4-methylbenzyl) oxalate, di(4-chlorobenzyl) oxalate, dimethyl phthalate, dimethyl terephthalate, dibenzyl terephthalate, dibenzyl isophthalate, bis(t-butylphenols); diphenylsulfone and derivatives thereof; diethers of 4,4'-dihydroxy diphenylsulfone such as 4,4'-dimethoxy diphenylsulfone, 4,4'-diethoxy diphenylsulfone, 4,4'-dipropoxy diphenylsulfone, 4,4'-diisopropoxy diphenylsulfone, 4,4'-dibutoxy diphenylsulfone, 4,4'-diisobutoxy diphenylsulfone, 4,4'-dipentyloxy diphenylsulfone, and 4,4'-dihexyloxy diphenylsulfone; diethers of 2,4'-dihydroxy diphenylsulfone such as 2,4'-dimethoxy diphenylsulfone, 2,4'-diethoxy diphenylsulfone, 2,4'-dipropoxy diphenylsulfone, 2,4'-diisopropoxy diphenylsulfone, 2,4'-dibutoxy diphenylsulfone, 2,4'-diisobutoxy diphenylsulfone, 2,4'-dipentyloxy diphenylsulfone, and 2,4'-dihexyloxy diphenylsulfone; 1,2-bis(phenoxy)ethane, 1,2-bis(4-methylphenoxy)ethane, 1,2-bis(3-methylphenoxy)ethane, 2-naphthol benzyl ether, diphenylamine, carbazole, 2,3-di-m-tolylbutane, 4-benzylbiphenyl, 4,4'-dimethylbiphenyl, m-terphenyl, di-β-naphthyl phenylenediamine, 1-hydroxy-naphthoic acid phenyl, 2-naphthyl benzyl ether, 4-methylphenyl-biphenyl ether, 2,2-bis(3,4-dimethylphenyl)ethane, 2,3,5,6-tetramethyl-4'-methyldiphenylmethane, diphenylcarbonate, and the like can be mentioned.

As the fillers, for example, silica, clay, kaoline, calcined kaoline, talc, satin white, aluminium hydroxide, calcium carbonate, magnesium carbonate, zinc oxide, titanium oxide, barium sulfate, magnesium silicate, aluminium silicate, plastic pigments or the like can be used. Specifically, alkaline earth metal salts are preferred in the present invention. More specifically, carbonates are preferred, and calcium carbonate and magnesium carbonate are even more specifically preferred. The ratio of the fillers used is 0.1 to 15 parts by weight, preferably 1 to 10 parts by weight, relatively to one part by weight of the color forming dye. Moreover, the fillers described above can be used by mixing together.

As the dispersants, for example, sulfosuccinic acid esters such as dioctyl sodium sulfosuccinate, sodium dodecylbenzene sulfonate, sodium salts of lauryl alcohol sulfuric esters, fatty acid salts, and the like can be mentioned.

As the antioxidants, for example, 2,2'-methylenebis(4-methyl-6-t-butylphenol), 2,2'-methylenebis(4-ethyl-6-t-butylphenol), 4,4'-propylmethylenebis(3-methyl-6-t-butylphenol), 4,4'-butylidenebis(3-methyl-6-t-butylphenol), 4,4'-thiobis(2-t-butyl-5-methylphenol), 1,1,3-tris(2-methyl-4-hydroxy-5-t-butylphenyl)butane, 1,1,3-tris(2-methyl-4-hydroxy-5-cyclohexylphenyl)butane, 4-{4-[1,1-bis(4-hydroxyphenyl)ethyl]-α,α-dimethylbenzyl}phenol, and the like can be mentioned.

As desensitizers, for example, aliphatic higher alcohols, polyethylene glycol, guanidine derivatives, and the like can be mentioned.

As the surface lubricants, for example, stearic acid, zinc stearate, calcium stearate, carnauba wax, paraffin wax, ester wax, and the like can be mentioned.

As the photostabilizers, for example, salicylic acid base ultraviolet-absorbers such as phenyl salicylate, p-t-butylphenyl salicylate, and p-octylphenyl salicylate; benzophenone base ultraviolet-absorbers such as 2,4-dihydroxybenzophenone, 2-hydroxy-4-methoxybenzophenone, 2-hydroxy-4-benzyloxybenzophenone, 2-hydroxy-4-octyloxybenzophenone, 2-hydroxy-4-dodecyloxybenzophenone, 2,2'-dihydroxy-4-methoxybenzophenone, 2,2'-dihydroxy-4,4'-dimethoxybenzophenone, 2-hydroxy-4-methoxy-5-sulfobenzophenone, and bis(2-methoxy-4-hydroxy-5-benzoylphenyl)methane; benzotriazole base ultraviolet-absorbers such as 2-(2'-hydroxy-5'-methylphenyl)benzotriazole, 2-(2'-hydroxy-5'-t-butylphenyl)benzotriazole, 2-(2'-hydroxy-3',5'-di-t-butylphenyl)benzotriazole, 2-(2'-hydroxy-3'-t-butyl-5'-methylphenyl)-5-chlorobenzotriazole, 2-(2'-hydroxy-3',5'-di-t-butylphenyl)-5-chlorobenzotriazole, 2-(2'-hydroxy-3',5'-di-t-amylphenyl)benzotriazole, 2-[2'-hydroxy-3'-(3",4",5",6"-tetrahydrophthalimide methyl)-5'-methylphenyl]benzotriazole, 2-(2'-hydroxy-5'-t-octylphenyl)benzotriazole, 2-[2'-hydroxy-3',5'-bis(α,α-dimethylbenzyl)phenyl]-2H-benzotriazole, 2-(2'-hydroxy-3'-dodecyl-5'-methylphenyl)benzotriazole, 2-(2'-hydroxy-3'-undecyl-5'-methylphenyl)benzotriazole, 2-(2'-hydroxy-3'-tridecyl-5'-methylphenyl)benzotriazole, 2-(2'-hydroxy-3'-tetradecyl-5'-methylphenyl)benzotriazole, 2-(2'-hydroxy-3'-pentadecyl-5'-methylphenyl)benzotriazole, 2-(2'-hydroxy-3'-hexadecyl-5'-methylphenyl)benzotriazole, 2-[2'-hydroxy-4'-(2"-ethylhexyl)oxyphenyl]benzotriazole, 2-[2'-hydroxy-4'-(2"-ethylheptyl)oxyphenyl]benzotriazole, 2-[2'-hydroxy-4'-(2"-ethyloctyl)oxyphenyl]benzotriazole, 2-[2'-hydroxy-4'-(2"-propyloctyl)oxyphenyl]benzotriazole, 2-[2'-hydroxy-4'-(2"-propylheptyl)oxyphenyl]benzotriazole, 2-[2'-hydroxy-4'-(2"-propylhexyl)oxyphenyl]benzotriazole, 2-[2'-hydroxy-4'-(1"-ethylhexyl)oxyphenyl]benzotriazole, 2-[2'-hydroxy-4'-(1"-ethylheptyl)oxyphenyl]benzotriazole, 2-[2'-hydroxy-4'-(1'-ethyloctyl)oxyphenyl]benzotriazole, 2-[2'-hydroxy-4'-(1"-propyloctyl)oxyphenyl]benzotriazole, 2-[2'-hydroxy-4'-(1"-propylheptyl)oxyphenyl]benzotriazole, 2-[2'-hydroxy-4'-(1"-propylhexyl)oxyphenyl]benzotriazole, and condensation products of polyethylene glycol and methyl-3-[3-t-butyl-5-(2H-benzotriazole-2-yl)-4-hydroxyphenyl]propionate; cyanoacrylate base ultraviolet-absorbers such as 2'-ethylhexyl-2-cyano-3,3-diphenylacrylate, and ethyl-2-cyano-3,3-diphenylacrylate; hindered amine base ultraviolet-absorbers such as bis(2,2,6,6-tetramethyl-4-piperidyl)sebacate, succinic acid-bis(2,2,6,6-tetramethyl-4-piperidyl)ester, and 2-(3,5-di-t-butyl) malonic acid-bis(1,2,2,6,6-pentamethyl-4-piperidyl)ester; 1,8-dihydroxy-2-acetyl-3-methyl-6-methoxynaphthalene, and the like can be mentioned.

As the fluorescent dyes, for example, 4,4'-bis[2-anilino-4-(2-hydroxyethyl)amino-1,3,5-triazinyl-6-amino]stilbene-2,2'-disulfonic acid=disodium salt, 4,4'-bis[2-anilino-4-bis(hydroxyethyl)amino-1,3,5-triazinyl-6-amino]stilbene-2,2'- disulfonic acid=disodium salt, 4,4'-bis[2-methoxy-4-(2-hydroxyethyl)amino-1,3,5-triazinyl-6-amino]stilbene-2,2'-disulfonic acid=disodium salt, 4,4'-bis[2-methoxy-4-(2-hydroxypropyl)amino-1,3,5-triazinyl-6-amino]stilbene-2,2'-disulfonic acid=disodium salt, 4,4'-bis[2-methoxy-4-(2-hydroxypropyl)amino-1,3,5-triazinyl-6-amino]stilbene-2,2'-disulfonic acid=disodium salt, 4,4'-bis[2-m-sulfoanilino-4-bis(hydroxyethyl)amino-1,3,5-triazinyl-6-amino]stilbene-2,2'-disulfonic acid=disodium salt, 4-[2-p-sulfoanilino-4-bis(hydroxyethyl)amino-1,3,5-triazinyl-6-amino]-4'-[2-m-sulfoanilino-4-bis(hydroxyethyl)amino-1,3,5-triazinyl-6-amino]stilbene-2,2'-disulfonic acid=tetrasodium salt, 4,4'-bis[2-p-sulfoanilino-4-bis(hydroxyethyl)amino-1,3,5-triazinyl-6-amino] stilbene-2,2'-disulfonic acid=tetrasodium salt, 4,4'-bis[2-(2,5-disulfoanilino)-4-phenoxyamino-1,3,5-triazinyl-6-amino]stilbene-2,2'-disulfonic acid=hexasodium salt, 4,4'-bis[2-(2,5-disulfoanilino)-4-(p-methoxycarbonylphenoxy)amino-1,3,5-triazinyl-6-amino] stilbene-2,2'-disulfonic acid=hexasodium salt, 4,4'-bis[2-(p-sulfophenoxy)-4-bis(hydroxyethyl)amino-1,3,5-triazinyl-6-amino]stilbene-2,2'-disulfonic acid=tetrasodium salt, 4,4'-bis[2-(2,5-disulfoanilino)-4-formalinylamino-1,3,5-triazinyl-6-amino]stilbene-2,2'-disulfonic acid=hexasodium salt, 4,4'-bis[2-(2,5-disulfoanilino)-4-bis(hydroxyethyl)amino-1,3,5-triazinyl-6-amino]stilbene-2,2'-disulfonic acid=hexasodium salt, and the like can be mentioned.

When the compound of the present invention is used for pressure-sensitive copying paper, it can be used in a manner similar to that of the known developer or sensitizer used for preparing the pressure-sensitive copying paper. For example, a color forming dye microencapsulated according to a known method is dispersed by using an appropriate dispersant, and is then coated on paper to prepare a color forming sheet. Moreover, dispersion liquid of the developer is applied on paper to prepare a developer sheet. At that time, if the compound of the present invention is used as an image storage stabilizer, it may be used by dispersing into either the dispersion liquid for the color forming sheet or that for the developer sheet. In this manner, both kinds of the sheets are combined together to prepare the pressure-sensitive copying paper. The pressure-sensitive copying paper may be a unit comprising an upper paper having an under surface to which is applied the microcapsules containing the organic solvent solution of the color forming dye, and an lower paper having a top surface to which is applied the developer (acid substance), or may be a so-called self-content paper having a surface on which the microcapsules and the developer are applied together.

As the developer used at that time or developers which may be used together with the compounds of the present invention, developers known in the prior art, for example, inorganic acid substances such as acid clay, activated clay, apatalgite, bentonite, colloidal silica, aluminum silicate, magnesium silicate, zinc silicate, tin silicate, calcined kaolin, and talc; aliphatic carboxylic acids such as oxalic acid, maleic acid, tartaric acid, citric acid, succinic acid, and stearic acid; aromatic carboxylic acids such as benzoic acid, p-t-butylbenzoic acid, phthalic acid, gallic acid, salicylic acid, 3-isopropyl salicylic acid, 3-phenyl salicylic acid, 3-cyclohexyl salicylic acid, 3,5-di-t-butyl salicylic acid, 3-methyl-5-benzyl salicylic acid, 3-phenyl-5-(2,2-dimethylbenzyl) salicylic acid, 3,5-di-(2-methylbenzyl) salicylic acid, and 2-hydroxy-1-benzyl-3-naphthoic acid, and metallic salts of these aromatic carboxylic acids, such as zinc salts, magnesium salts, aluminum salts, and titanium salts; phenol resin base developers such as p-phenylphenol-formalin resin, and p-butylphenol-acetylene resin, and mixtures of these phenol resin base developers and the aforementioned metallic salts of the aromatic carboxylic acids can be mentioned.

As the supports used for the present invention, papers known in the prior art, synthetic papers, films, plastic films, foaming plastic films, nonwoven fabrics, recycled papers such as used pulp papers and the like can be used. Moreover, these may be combined together to use as the supports.

Moreover, the compound represented by the formula (I) of the present invention may be used as a developer of a polychrome heat-sensitive recording material having a high-temperature color forming layer and at least one low-temperature color forming layer which takes on a color different from that of the high-temperature color forming layer at lower temperature than that of the high-temperature color forming layer. In this case, the compound represented by the formula (I) can be used as a developer for the high-temperature color forming layer as well as that for the low-temperature color forming layer.

### Best Mode for Carrying Out the Invention

In the following, the recording material of the present invention will be explained in detail with examples, but the present invention is not limited to these. Note that parts shown hereinafter are based on weight.

### Synthesis Example 1

### Synthesis of methyl 4-(4-hydroxyphenylsulfonyl)phenoxyacetate (Compound No. 1)

50 g (0.2 mol) of 4,4'-dihydroxy diphenylsulfone, 27.6 g (0.2 mol) of potassium carbonate, and 100 ml of dimethylsulfoxide were charged into a 300 ml four-necked flask equipped with a stirrer and a thermometer at room temperature, and then were heated to 50 °C. After that, 21.7 g (0.2 mmol) of methyl chloroacetate was added, and then was reacted for 5 hours at 50 to 60 °C. After the end of the reaction, 200 ml of water was added, and then extraction was carried out with MIBK. After a MIBK layer was washed several times with 1 % sodium carbonate solution to remove unreacted 4,4'-dihydroxy diphenylsulfone, MIBK was evaporated under reduced pressure, and then precipitated crystals were filtrated off, followed by being recrystallized with acetone and chloroform to obtain 35.0 g of methyl 4-(4-hydroxyphenylsulfonyl)phenoxyacetate. The yield was 54 % and the melting point was 159 to 161 °C.

### Synthesis Example 2

### Synthesis of 2-hydroxyethyl 4-(4-hydroxyphenylsulfonyl)phenoxyacetate (Compound No. 3)

2-hydroxyethyl 4-(4-hydroxyphenylsulfonyl)phenoxyacetate was obtained in the same way as that of Synthesis Example 1 except that methyl chloroacetate in Synthesis Example 1 was replaced by 2-hydroxyethyl chloroacetate.

### Synthesis Example 3

### Synthesis of 2-phenoxyethyl 4-(4-hydroxyphenylsulfonyl)phenoxyacetate (Compound No. 5)

2-phenoxyethyl 4-(4-hydroxyphenylsulfonyl)phenoxyacetate was obtained in the same way as that of Synthesis Example 1 except that methyl chloroacetate in Synthesis Example 1 was replaced by 2-phenoxyethyl chloroacetate.

### Synthesis Example 4

### Synthesis of n-propyl 4-(4-hydroxyphenylsulfonyl)phenoxyacetate (Compound No. 6)

N-propyl 4-(4-hydroxyphenylsulfonyl)phenoxyacetate was obtained in the same way as that of Synthesis Example 1 except that methyl chloroacetate in Synthesis Example 1 was replaced by n-propyl chloroacetate.

### Synthesis Example 5

### Synthesis of isopropyl 4-(4-hydroxyphenylsulfonyl)phenoxyacetate (Compound No. 7)

Isopropyl 4-(4-hydroxyphenylsulfonyl)phenoxyacetate was obtained in the same way as that of Synthesis Example 1 except that methyl chloroacetate in Synthesis Example 1 was replaced by isopropyl chloroacetate.

### Synthesis Example 6

### Synthesis of benzyl 4-(4-hydroxyphenylsulfonyl)phenoxyacetate (Compound No. 10)

Benzyl 4-(4-hydroxyphenylsulfonyl)phenoxyacetate was obtained in the same way as that of Synthesis Example 1 except that methyl chloroacetate in Synthesis Example 1 was replaced by benzyl chloroacetate.

### Synthesis Example 7

### Synthesis of methyl 2-[4-(4-hydroxyphenylsulfonyl)phenoxy]propionate (Compound No. 13)

Methyl 2-[4-(4-hydroxyphenylsulfonyl)phenoxy]propionate was obtained in the same way as that of Synthesis Example 1 except that methyl chloroacetate in Synthesis Example 1 was replaced by methyl 2-chloropropionate.

### Synthesis Example 8

### Synthesis of methyl 2-[4-(4-hydroxyphenylsulfonyl)phenoxy]isobutyrate (Compound No. 25)

Methyl 2-[4-(4-hydroxyphenylsulfonyl)phenoxy]isobutyrate was obtained in the same way as that of Synthesis Example 1 except that methyl chloroacetate in Synthesis Example 1 was replaced by methyl 2-chloroisobutyrate.

### Synthesis Example 9

### Synthesis of ethyl 2-[4-(4-hydroxyphenylsulfonyl)phenoxy]isobutyrate (Compound No. 26)

Ethyl 2-[4-(4-hydroxyphenylsulfonyl)phenoxy]isobutyrate was obtained in the same way as that of Synthesis Example 1 except that methyl chloroacetate in Synthesis Example 1 was replaced by ethyl 2-chloroisobutyrate.

### Synthesis Example 10

### Synthesis of 4-(4-hydroxyphenylsulfonyl)phenoxyacetic acid diethylamide (Compound No. 56)

30 g (0.12 mol) of 4,4'-dihydroxy diphenylsulfone was charged into a 500 ml four-necked flask equipped with a stirrer and a thermometer, into which 50 ml of acetonitrile and 50 ml of dimethyl sulfoxide were added at room temperature to produce a homogeneous solution. After 8.3 g (0.06 mol) of potassium carbonate was added into that at room temperature, 12.5 g (0.08 mol) of chloroacetic acid dietylamide was added, and then was reacted for 5 hours at 70 °C. After the end of the reaction, 200 ml of water was added, and then an extraction process was carried out with 200 ml of MIBK. After a MIBK layer was subjected twice to extraction with 200ml of 15 % NaOH solution, a water layer was adjusted to pH 4, from which precipitated crystals were filtrated. By washing the crystals with methanol and acetone, 30.5 g of 4-(4-hydroxyphenylsulfonyl)phenoxyacetic acid diethylamide was obtained. The yield was 70 %. The melting point was 223 to 226 °C.

### Synthesis Example 11

### Synthesis of 4-(4-hydroxyphenylsulfonyl)phenoxyacetic acid di(n-butyl)amide (Compound No. 59)

4-(4-hydroxyphenylsulfonyl)phenoxyacetic acid di(n-butyl)amide was obtained in the same way as that of Synthesis Example 10 except that chloroacetic acid dietylamide in Synthesis Example 10 was replaced by chloroacetic acid di(n-butyl)amide.

### Example 1 (Preparation of heat-sensitive recording paper)

| Dye dispersion (A solution) | |
|---|---|
| 3-di-n-butylamino-6-methyl-7-anilinofluoran | 16 parts |
| 10% aqueous solution of polyvinyl alcohol | 84 parts |

| Developer dispersion (B solution) | |
|---|---|
| Methyl 4-(4-hydroxyphenylsulfonyl)phenoxyacetate | 16 parts |
| 10% aqueous solution of polyvinyl alcohol | 84 parts |

| Filler dispersion (C solution) | |
|---|---|
| Calcium carbonate | 27.8 parts |
| 10% aqueous solution of polyvinyl alcohol | 26.2 parts |
| Water | 71 parts |

A coating solution was prepared by mixing together with 1 part by weight of A solution, 2 parts by weight of B solution, and 4 parts by weight of C solution, each of which was a dispersion solution prepared by sufficiently pulverizing a mixture of their respective components by the use of a sandgrinder. After this coating solution was coated on white paper by the use of a wire rod (No. 12) and then was dried, it was subjected to calendering treatment to produce heat-sensitive recording paper (the amount of application was about 5.5 g/m² by dry weight).

### Example 2

A heat-sensitive recording paper was prepared in the same way as that of Example 1 except that methyl 4-(4-hydroxyphenylsulfonyl)phenoxyacetate in the developer dispersion (B solution) of Example 1 was replaced by 2-hydroxyethyl 4-(4-hydroxyphenylsulfonyl)phenoxyacetate.

### Example 3

A heat-sensitive recording paper was prepared in the same way as that of Example 1 except that methyl 4-(4-hydroxyphenylsulfonyl)phenoxyacetate in the developer dispersion (B solution) of Example 1 was replaced by 2-phenoxyethyl 4-(4-hydroxyphenylsulfonyl)phenoxyacetate.

### Example 4

A heat-sensitive recording paper was prepared in the same way as that of Example 1 except that methyl 4-(4-hydroxyphenylsulfonyl)phenoxyacetate in the developer dispersion (B solution) of Example 1 was replaced by n-propyl 4-(4-hydroxyphenylsulfonyl)phenoxyacetate.

### Example 5

A heat-sensitive recording paper was prepared in the same way as that of Example 1 except that methyl 4-(4-hydroxyphenylsulfonyl)phenoxyacetate in the developer dispersion (B solution) of Example 1 was replaced by isopropyl 4-(4-hydroxyphenylsulfonyl)phenoxyacetate.

### Example 6

A heat-sensitive recording paper was prepared in the same way as that of Example 1 except that methyl 4-(4-hydroxyphenylsulfonyl)phenoxyacetate in the developer dispersion (B solution) of Example 1 was replaced by benzyl 4-(4-hydroxyphenylsulfonyl)phenoxyacetate.

### Example 7

A heat-sensitive recording paper was prepared in the same way as that of Example 1 except that methyl 4-(4-hydroxyphenylsulfonyl)phenoxyacetate in the developer dispersion (B solution) of Example 1 was replaced by methyl 2-[4-(4-hydroxyphenylsulfonyl)phenoxy]propionate.

### Example 8

A heat-sensitive recording paper was prepared in the same way as that of Example 1 except that methyl 4-(4-hydroxyphenylsulfonyl)phenoxyacetate in the developer dispersion (B solution) of Example 1 was replaced by methyl 2-[4-(4-hydroxyphenylsulfonyl)phenoxy]isobutyrate.

### Example 9

A heat-sensitive recording paper was prepared in the same way as that of Example 1 except that methyl 4-(4-hydroxyphenylsulfonyl)phenoxyacetate in the developer dispersion (B solution) of Example 1 was replaced by ethyl 2-[4-(4-hydroxyphenylsulfonyl)phenoxy]isobutyrate.

### Example 10

A heat-sensitive recording paper was prepared in the same way as that of Example 1 except that methyl 4-(4-hydroxyphenylsulfonyl)phenoxyacetate in the developer dispersion (B solution) of Example 1 was replaced by 4-(4-hydroxyphenylsulfonyl)phenoxyacetic acid diethylamide.

### Example 11

A heat-sensitive recording paper was prepared in the same way as that of Example 1 except that methyl 4-(4-hydroxyphenylsulfonyl)phenoxyacetate in the developer dispersion (B solution) of Example 1 was replaced by 4-(4-hydroxyphenylsulfonyl)phenoxyacetic acid di(n-butyl)amide.

### Comparative Example 1

A heat-sensitive recording paper was prepared in the same way as that of Example 1 except that methyl 4-(4-hydroxyphenylsulfonyl)phenoxyacetate in the developer dispersion (B solution) of Example 1 was replaced by 4-hydroxy-4'-isopropoxy diphenylsulfone.

### Test Example 1 (Dynamic density)

The heat-sensitive recording papers prepared in Examples 1 to 11 and Comparative Example 1 were color-developed by using a heat-sensitive paper color-developing test apparatus (manufactured by OKURA Electric Co. Ltd., type TH-PMD) under the condition of 0.72 mj per one dot, and then their printing densities were measured by the use of a Macbeth reflectance densitometer (manufactured by Macbeth Co. Ltd., RD-514). Measurement results are shown in Table 3.

### Test Example 2 (Heat resistance examination of a background part)

One part of each of the heat-sensitive recording papers prepared in Examples 1 to 11 and Comparative Example 1 was cut off, and then was left in an incubator (type DK-400, manufactured by YAMATO) for 24 hours at 90 °C, 100 °C, 110 °C, or 120 °C.

After that, their background optical densities (Macbeth values) were measured. Measurement results are shown in Table 3. As is obvious from Table 3, each of the heat-sensitive recording papers prepared in Examples 1 to 11 has a heat resistance of a background part, exceedingly superior to that of Comparative Example 1, even under the high-temperature conditions at 110 °C and 120 °C.

### Test Example 3 (Heat and humidity resistance examination of a background part)

One part of each of the heat-sensitive recording papers prepared in Examples 1 to 11 and Comparative Example 1 was cut off, and then was left in an incubator (type GL-42, manufactured by Futaba Kagaku Co. Ltd.,) at 50°C and 80% humidity for 2 hours or 24 hours.

After that, their background optical densities (Macbeth values) were measured. Measurement results are shown in Table 3. As is obvious from Table 3, each of the heat-sensitive recording papers prepared in Examples 1 to 11 has a heat and humidity resistance of a background part, superior to that of Comparative Example 1.

**Table 3**

| (Evaluation test results) | | | | | | | | |
|---|---|---|---|---|---|---|---|---|
| | Orginal background part | Heat and humidity resistance of background part | | Heat resistance of background part (°C) | | | | Image density (0.72 mj/dot) |
| | | 2 hr | 24 hr | 90 | 100 | 110 | 120 | |
| Ex. 1 | 0.06 | 0.06 | 0.05 | 0.06 | 0.07 | 0.09 | 0.16 | 1.32 |
| Ex. 2 | 0.05 | 0.04 | 0.04 | 0.05 | 0.05 | 0.05 | 0.06 | 1.14 |
| Ex. 3 | 0.05 | 0.04 | 0.04 | 0.12 | 0.17 | 0.40 | 1.06 | 1.19 |
| Ex. 4 | 0.05 | 0.05 | 0.04 | 0.07 | 0.17 | 0.56 | 1.24 | 1.33 |
| Ex. 5 | 0.05 | 0.04 | 0.04 | 0.06 | 0.07 | 0.12 | 0.35 | 1.34 |
| Ex. 6 | 0.04 | 0.04 | 0.04 | 0.05 | 0.05 | 0.05 | 0.07 | 1.25 |
| Ex. 7 | 0.05 | 0.05 | 0.05 | 0.05 | 0.06 | 0.07 | 0.11 | 1.29 |
| Ex. 8 | 0.04 | 0.04 | 0.04 | 0.04 | 0.05 | 0.07 | 0.12 | 1.32 |
| Ex. 9 | 0.04 | 0.04 | 0.04 | 0.04 | 0.05 | 0.06 | 0.11 | 1.34 |
| Ex. 10 | 0.04 | 0.04 | 0.04 | 0.04 | 0.04 | 0.05 | 0.06 | 0.92 |
| Ex. 11 | 0.04 | 0.04 | 0.04 | 0.05 | 0.05 | 0.05 | 0.07 | 0.84 |
| C. Ex. 1 | 0.08 | 0.09 | 0.08 | 0.14 | 0.28 | 1.11 | 1.24 | 1.30 |

### Industrial Applicability

As described above, the recording materials exceeding in the storage stability of their background parts, specifically in the heat resistance and the heat and humidity resistance of the background parts, and the novel diphenylsulfone derivative compounds were provided according to the present invention.

## Claims

1. A recording material including a color forming dye, comprising: at least one of diphenylsulfone derivatives represented by the general formula (I): 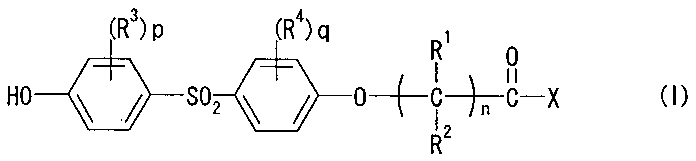 (wherein R¹ and R² each independently represents a hydrogen atom or a C1 to C6 alkyl group, n represents an integer of 1 to 6, R³ and R⁴ each independently represents a halogen atom, a C 1 to C6 alkyl group, a C2 to C6 alkenyl group, or a C1 to C6 alkoxy group, p and q each independently represents an integer of 0 to 4, R³ and R⁴ may be the same or different when p and q each represents an integer of 2 or more, X represents OR⁵ or NR⁶R⁷ wherein R⁵ represents a C1 to C6 alkyl group, a C1 to C6 hydroxyalkyl group, a C1 to C6 alkoxy-C1 to C6 alkyl group, a phenoxy-C1 to C6 alkyl group, a phenyl group which may have a substituent, or a benzyl group which may have a substitutent, and R⁶ and R⁷ each independently represents a hydrogen atom, a C1 to C6 alkyl group, a phenyl group which may have a substituent, or a benzyl group which may have a substituent).

2. A diphenylsulfone derivative represented by the general formula (II): 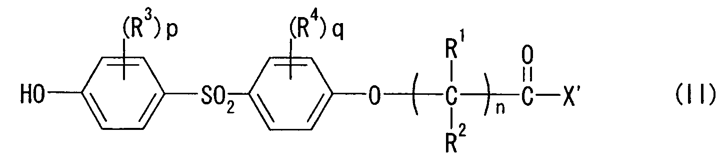 (wherein R¹ and R² each independently represents a hydrogen atom or a C1 to C6 alkyl group, n represents an integer of 1 to 6, R³ and R⁴ each independently represents a halogen atom, a C1 to C6 alkyl group, a C2 to C6 alkenyl group, or a C1 to C6 alkoxy group, p and q each independently represents an integer of 0 to 4, R³ and R⁴ may be the same or different when p and q each represents an integer of 2 or more, X' represents OR⁸ or NR⁶R⁷ wherein R⁸ represents a C1 to C6 alkyl group, a C1 to C6 hydroxyalkyl group, a C1 to C6 alkoxy-C1 to C6 alkyl group, a phenoxy-C1 to C6 alkyl group, a phenyl group which may have a substituent, or a benzyl group which may have a substitutent, and R⁶ and R⁷ each independently represents a hydrogen atom, a C1 to C6 alkyl group, a phenyl group which may have a substituent, or a benzyl group which may have a substituent, provided that R⁸ is not an ethyl group when R¹ represents a hydrogen atom, R² represents a hydrogen atom, n represents 1, p represents 0, q represents 0, and X' represents OR⁸).
